# EUROPEAN PATENT SPECIFICATION

(11) **EP 3 382 109 B1**
(45) Date of publication and mention of the grant of the patent: **18.05.2022**
(21) Application number: 18163517.8
(22) Date of filing: 23.03.2018
(51) Int. Cl.: E02F 9/22, E02F 9/26, F15B 21/04, G01N 11/00, F15B 21/041, G01N 33/28

(54) **WORK VEHICLE HYDRAULIC CIRCUIT WITH HYDRAULIC FLUID CONDITION MONITORING SENSOR**
HYDRAULIKKREIS FÜR EINE ARBEITSMASCHINE MIT EINEM HYDRAULIKFLUIDÜBERWACHUNGSSENSOR
CIRCUIT HYDRAULIQUE POUR UN ENGIN DE TRAVAIL COMPORTANT UN CAPTEUR DE CONDITION DU FLUIDE HYDRAULIQUE

(30) Priority: 27.03.2017 IT 201700033036
(43) Date of publication of application: 03.10.2018
(73) Proprietor: CNH Industrial Italia S.p.A., 10135 Torino (IT)
(72) Inventor: BENEVELLI, Alessandro, 42020 Albinea, Reggio Nell'Emilia (IT); FERRARI, Luca, 41043 Formigine (Modena) (IT); ODDO, Antonio, 10135 Torino (IT); POSSELIUS, John H., Ephrata, PA 17522 (US)
(74) Representative: CNH Industrial IP Department

(56) References cited:
- CN-A- 101 865 179
- JP-A- H06 117 987
- US-A1- 2004 128 107
- US-A1- 2007 222 573
- US-A1- 2015 027 569
- US-A1- 2016 230 785

## Description

### BACKGROUND OF THE INVENTION

The present invention relates to monitoring hydraulic oil quality in an agricultural tractor or other agricultural, industrial, commercial, or construction vehicle, and, more specifically to preventing contamination of the hydraulic oil of the agricultural tractor or other agricultural, industrial, commercial, or construction vehicle when such vehicle is used in conjunction with an implement, device, or attachment.

Farmers utilize a wide variety of agricultural implements or devices to perform agricultural functions. Often, these agricultural implements or devices are provided with functions that are actuated by hydraulics, which receive hydraulic pressure and flow from the agricultural tractor or machine to which the agricultural implement is attached. A given farming operation may use multiple agricultural implements or devices and multiple agricultural tractors or machines interchangeably. As a result, a given agricultural implement or device may be variously hydraulically connected to multiple agricultural tractors or machines, and a given agricultural tractor or machine may be variously hydraulically connected to multiple agricultural implements or devices. This interchangeability may extend to using agricultural tractors or machines and agricultural implements or devices from different manufacturers. Similarly, commercial vehicle operators may utilize a wide variety of vehicles interchangeably with multiple attachments.

When an agricultural implement or device is disconnected from an agricultural tractor or machine, or when an attachment is disconnected from an industrial, commercial, or construction vehicle, the connectors are designed so that the hydraulic oil does not leak out of either. A large volume of hydraulic oil therefore remains within the implement, device, or attachment. This hydraulic oil then becomes intermixed with the hydraulic oil of the next agricultural tractor or machine, or industrial, commercial, or construction vehicle, to which the implement, device, or attachment is connected. Over time, the hydraulic oil within a given implement, device, or attachment may deteriorate, become contaminated, or otherwise degrade. When the given implement, device, or attachment is then connected to another agricultural tractor, machine, or industrial, commercial, or construction vehicle, the deteriorated, contaminated, or otherwise degraded hydraulic oil may thereby contaminate the hydraulic oil of the agricultural tractor or machine, or industrial, commercial, or construction vehicle.

In addition, in the process of connecting the hoses of the implement, device, or attachment to the agricultural tractor, machine, or industrial, commercial, or construction vehicle, foreign contaminants may be introduced into the hydraulic circuits of either, due to dirt, dust, and contamination present on the surfaces of the hydraulic hoses and connectors. Further, it may also happen that an implement, device, or attachment being connected to an agricultural tractor, machine, or industrial, commercial, or construction vehicle has hydraulic oil that is of an incompatible viscosity or other characteristic, either according to the specifications of the implement, device, or attachment, or as a result of degradation.

In any of these situations, intermixing of the deteriorated, contaminated, degraded, or incompatible hydraulic oil from the implement, device, or attachment with the hydraulic oil of the agricultural tractor, machine, or industrial, commercial, or construction vehicle can result in damage or unwanted wear to the hydraulic system components of the agricultural tractor, machine, or industrial, commercial, or construction vehicle. In order to minimize this effect, it is known to provide agricultural tractors, machines, or industrial, commercial, or construction vehicles with large hydraulic reservoirs, thereby minimizing the diluting effect of the hydraulic oil within the implement, device, or attachment. It is further known to provide filtration systems, in order to remove contaminants from the hydraulic oil. However, despite these measures, intermixing of deteriorated, contaminated, degraded, or incompatible hydraulic oil from an implement, device, or attachment with the hydraulic oil of an agricultural tractor, machine, or industrial, commercial, or construction vehicle, continues to be a source of problems for farmers and industrial, commercial, or construction vehicle operators.

U.S. Published Application No. 2007/0222573 (Navarro et al.) attempts to avoid damage or unwanted wear to the hydraulic system components of a work vehicle by providing a method and device for determining the appropriate time to recharge hydraulic fluid in the work vehicle. The method ... includes making a real time determination of at least a quality parameter of hydraulic fluid in a work vehicle. The system includes comparing in real time the quality parameter to a predetermined value for the hydraulic fluid. The method ... also includes a system for comparing the determined values and communicating [them] to an operator. The method ... also includes the capacity to control the output level of the work vehicle according to the level of contamination in the hydraulic fluid. [It] ... also provides a device for real time monitoring and control of the hydraulic fluid. (Abstract).

The system ... includes a sensor or sensors operably associated or connected to a work vehicle engine and a controller. (Para. [0008]).... the quality parameters of interest for the work vehicle may include particle count, viscosity, water saturation and oxidation, and temperature of the hydraulic fluid in the work vehicle.... The particle count detector is ... incorporated into the hydraulic system at strategic locations such as along fluid lines with results transmitted to an instrument control panel or controller for operator's use.... Other quality parameters such as viscosity, humidity, oxidation number, or total acid number may be useful in verifying or corroborating the particle count detector determination. (Para. [0014]).

When the sensors determine the presence of new or recharged hydraulic fluid in the work vehicle, a determination of a quality parameter of interest may then be accomplished ... if the quality parameter of interest is outside the specification for such work vehicle, ..., an alert is preferably transmitted to the controller. If the parameters of interest are still acceptable or within the specification, the operation of the work vehicle continues without need to communicate an alert to the system. (Para. [0011]). When the quality parameter of interest is deemed critical, a power output control system ... may be activated. This power control system may progressively manage (by reducing or diminishing) the output capacity of the work vehicle engine, thus preventing a catastrophic failure due to need for a recharge of hydraulic fluid.... The power control system may disable the work vehicle if deemed appropriate to prevent damage. (Para. [0013]).

However, Navarro et al. does not prevent intermixing of deteriorated, contaminated, degraded, or incompatible hydraulic oil from an implement, device, or attachment with the hydraulic oil of an agricultural tractor, machine, or industrial, commercial, or construction vehicle. Navarro et al. merely alerts the system and/or the operator to the parameters of the hydraulic oil already within the hydraulic system of the tractor being outside the specification of the work vehicle, and limits the output capacity of the work vehicle engine to prevent damage. By that point, the hydraulic oil of the agricultural tractor, machine, or industrial, commercial, or construction vehicle has already become contaminated, and may need to be replaced. This represents a costly and time consuming waste for the farmer or commercial vehicle operator. Further, damage to the hydraulic systems of the agricultural tractor, machine, or industrial, commercial, or construction vehicle may have already occurred.

Similarly, U.S. Published Application No. 2004/0128107 (Ryu et al.) diagnoses contamination of hydraulic oil after the fact.... a sensor value concerning an oil pollution state is measured by installing a sensor unit in a hydraulic system in the interior of a [piece of] heavy equipment, and a result value is computed from the sensor value by a diagnosis program, ..., for thereby enhancing [the] durability and lifespan of [the piece of] heavy equipment ... (Abstract).... in [a piece of] heavy equipment having a hydraulic circuit line which is formed of a hydraulic pump, a hydraulic motor, a major adjusting valve, a remote adjusting valve, a pipe, a hose, a hydraulic tank and an oil cooler, there is provided ... an oil pollution degree diagnosis function ... comprising a sensor unit which is installed in such a manner that ... oil is flown into the interior of the same ... and is adapted to extract ... oil flowing in the hydraulic circuit and to measure an oil pollution state sensor value... (Para. [0012]). The sensor unit 221 is installed in a bypass of an oil cooler in a process that ... oil is flown into an oil tank through a hydraulic pump, MCV and hydraulic actuator in a hydraulic tank in the hydraulic circuit system of the [piece of] heavy equipment. In addition, ... the sensor unit 222 ... is installed in an input line of the oil cooler, the sensor unit 223 ... is installed in an input and output side of a return filter, and the sensor unit 224 ... is installed in a bypass line. (Para. [0038]).

Again, Ryu et al. does not prevent intermixing of deteriorated, contaminated, degraded, or incompatible hydraulic oil from an implement, device, or attachment with the hydraulic oil of an agricultural tractor, machine, or industrial, commercial, or construction vehicle. Ryu et al merely transmits the sensor results to an oil pollution diagnosis server, and eventually displays this information on a display unit of the piece of heavy equipment. Again, by that point, the hydraulic oil of the agricultural tractor, machine, or industrial, commercial, or construction, vehicle may have already become contaminated, and may need to be replaced, once more representing a costly and time consuming waste for the farmer or equipment operator. Again, damage to the hydraulic systems of the agricultural tractor, machine, or industrial, commercial, or construction vehicle may have already occurred by this point.

U.S. Published Application No. 2014/0150304 (Sherlock et al.) attempts to provide for appropriately timed maintenance of hydraulic fluid and filters in a work vehicle and auxiliary tools by calculating the effective use time thereof. The hydraulic management system automatically calculates an effective use time of a hydraulic element, such as [the] hydraulic fluid or hydraulic filters, by multiplying work tool usage by a desired gain factor, where the gain factor may exceed 1 for auxiliary work tools. (Abstract). The controller increases the effective use time at a first rate based on usage of the first hydraulic work tool and at a second rate based on usage of the second hydraulic work tool, the second rate differing from the first rate. (Para. [0004]).... a hydraulic management system including a controller and a gain input that communicates a gain factor associated with the at least one hydraulic work tool to the controller. The controller multiplies usage of the at least one hydraulic work tool by the gain factor to determine an effective use time of at least one hydraulic element of the work vehicle. (Para. [0005]).

However, Sherlock et al. in no way takes into account the actual condition of the hydraulic fluid in the implement, device, or attachment, which may have been used by another agricultural tractor, machine, or industrial, commercial, or construction vehicle, or may otherwise been contaminated or have deteriorated in the interim.

What is needed in the art, therefore, is a system or arrangement implemented of hydraulics on an agricultural tractor or other agricultural, industrial, commercial, or construction vehicle that is regularly connected to multiple implements, devices, or attachments, which system or arrangement limits or prevents deteriorated, contaminated, or otherwise degraded hydraulic oil within the implement, device, or attachment from intermixing with the hydraulic oil of the agricultural tractor or other agricultural, industrial, commercial, or construction vehicle. JPH06117987 shows a hydraulic system according to the preamble of independent claim 1.

### SUMMARY OF THE INVENTION

Embodiments of the present invention provide such a system or arrangement of hydraulic circuits implemented on an agricultural tractor or other agricultural, industrial, commercial, or construction vehicle that limits or prevents deteriorated, contaminated, or otherwise degraded hydraulic oil within an implement, device, or attachment from intermixing with the hydraulic oil of the agricultural tractor or other agricultural, industrial, commercial, or construction vehicle. The system or arrangement according to various embodiments of the present invention limits or avoids such contamination by providing at least one hydraulic oil quality and/or viscosity sensor either within the hydraulic circuit of the implement, device, or attachment or directly at the c onnection(s) between the tractor, or other agricultural, industrial, commercial, or construction vehicle, and the implement, device, or attachment. These connections may be in the form of electro hydraulic remote connections, particularly in the case of agricultural tractors.

By having at least one hydraulic oil quality and/or viscosity sensor directly at the connections between the tractor, or other agricultural, industrial, commercial, or construction vehicle, and the implement, device, or attachment, embodiments of the present invention provide information to an operator and/or control systems of the tractor, or other agricultural, industrial, commercial, or construction vehicle, regarding the state of contamination, deterioration, degradation, viscosity, or other characteristic of the hydraulic oil within the implement, device, or attachment hydraulic circuits. The operator and/or control system of the tractor, or other agricultural, industrial, commercial, or construction vehicle may then react to the presence of contaminated, deteriorated, degraded hydraulic oil, or to the presence of hydraulic oil of improper viscosity or other characteristic, by shutting off, isolating, or disconnecting the implement, device, or attachment hydraulic circuits before significant degradation or contamination of the hydraulic oil within the hydraulic circuits of the tractor, or other agricultural, industrial, commercial, or construction vehicle, occurs.

Embodiments of the present invention utilize at least one hydraulic oil quality and/or viscosity sensor that is capable of measuring one or more parameters of the implement hydraulic oil, such as viscosity, dielectric constant, density, conductivity, relative humidity, moisture content, presence of metal or other contaminants, chemical makeup, temperature, and/or pressure. In this way, when the hydraulic circuits of the implement, device, or attachment are connected to the hydraulic circuits of the tractor or other agricultural, industrial, commercial, or construction vehicle by way of the electro hydraulic remote connections, where applicable, and when hydraulic oil within the implement, device, or attachment begins to flow through the electro hydraulic remote connections, the at least one hydraulic oil quality and/or viscosity sensor can communicate the one or more measured parameters of the implement hydraulic oil to the operator and/or control system, which control system may be in the form of an electronic control unit (ECU).

The ECU of certain embodiments of the present invention may then compare the one or more measured parameters of the implement hydraulic oil with one or more standard parameters for hydraulic oil, one or more absolute or comparative thresholds of acceptability for the one or more measured parameters, and/or known or measured parameters of the hydraulic oil within the tractor or other agricultural, industrial, commercial, or construction vehicle. A comparative threshold of acceptability of the one or more measured parameters may be determined by comparing the one or more measured parameters of the implement hydraulic oil with known or measured parameters of the hydraulic oil within the hydraulic circuits of the tractor or other agricultural, industrial, commercial, or construction vehicle, and determining if the one or more measured parameters are sufficiently close given the current condition of the hydraulic oil within the hydraulic circuits of the tractor or other agricultural, industrial, commercial, or construction vehicle. This is done in order to determine if the quality and/or viscosity or other characteristics of the hydraulic oil within the hydraulic circuits of the implement, device, or attachment are suitable to intermix with the hydraulic oil within the hydraulic circuits of the tractor or other agricultural, industrial, commercial, or construction vehicle, or whether there is one or more relevant outstanding difference in the one or more measured parameters of the implement hydraulic oil that warrants shutting off, isolating, or disconnecting the implement, device, or attachment hydraulic circuits, or otherwise alerting the operator.

As a non-limiting example, the ECU of a certain embodiment of the present invention may compare the parameter of viscosity of the hydraulic oil within the hydraulic circuits of an implement being connected to an agricultural tractor as measured by the at least one hydraulic oil quality and/or viscosity sensor located directly at the agricultural tractor's electro hydraulic remote connections with the known or measured viscosity of the hydraulic oil within the hydraulic circuits of the agricultural tractor. As it is detrimental to mix hydraulic oils with differing viscosities, if the ECU determines that the hydraulic oil within the hydraulic circuits of the implement is of a sufficiently different viscosity than the hydraulic oil within the hydraulic circuits of the agricultural tractor, the ECU commands one or more valves co-located with or integrated with the tractor electro hydraulic remote connections to close. This isolates the hydraulic oil within the hydraulic circuits of the agricultural tractor from the hydraulic oil of improper viscosity within the hydraulic circuits of the implement, thereby preventing contamination of the hydraulic oil of the agricultural tractor, and possibly preventing unwanted downtime and expenditure that would have been necessary to drain and replace the hydraulic oil in the agricultural tractor and/or in the implement.

The same principle may apply in various embodiments of the present invention wherein one or more of various parameters such as viscosity, dielectric constant, density, conductivity, relative humidity, moisture content, presence of metal or other contaminants, chemical makeup, temperature, and/or pressure are measured by the at least one hydraulic oil quality and/or viscosity sensor located at or integrated with the connections between the tractor, or other agricultural, industrial, commercial, or construction vehicle, and the implement, device, or attachment being connected thereto. Alternate embodiments of the present invention may command one or more valves co-located with or integrated with the hydraulic connection to close upon measurement of one or more detrimental parameters of the implement hydraulic oil, as described previously, or may simply alert an operator, or may both command one or more valves to close and alert the operator. The ECU may further cause a display to instruct the operator as to an appropriate action to be taken by the operator. Further alternate embodiments may command the one or more valves to close unless overridden by the operator, or may command the one or more valves to close upon verification by the operator.

Yet further alternate embodiments may command the one or more valves to remain closed by default upon connection of the implement, device, or attachment to the agricultural, industrial, commercial, or construction vehicle, until such time that the at least one hydraulic oil quality and/or viscosity sensor determines that the one or more of parameters of interest, such as viscosity, dielectric constant, density, conductivity, relative humidity, moisture content, presence of metal or other contaminants, chemical makeup, temperature, and/or pressure, are within acceptable limits.

Embodiments of the present invention may use one or more commercially available hydraulic oil quality and/or viscosity sensors, such as:
- Fluid Property Sensor model number FPS2800B12C4 available from TE Sensor Solutions, Toulouse, France, which measures viscosity, density, dielectric constant, and temperature, which is capable of < 25 bar and 150 degrees C maximum, and which is compatible with a CAN interface;
- HYDAC Lab model 1400 available from HYDAC, Glendale Heights, Illinois, which measures dielectric constant, temperature, and relative humidity, which is capable of maximum flow of 5 m/s, < 10 bar, and 80 degrees C maximum, and which is compatible with 3 channel analogue;
- LubCos Vis available from Argo Hytos, Bowling Green, Ohio, which measures viscosity, dielectric constant, and temperature, which is capable of < 50 bar and 85 degrees C maximum, and which is compatible with a CAN or RS232 interface;
- OPCom FerroS available from Argo Hytos, Bowling Green, Ohio, which measures ferromagnetic wear particles, and which is capable of 0.05 m/s minimum flow, 1 m/s maximum flow, < 20 bar, and 85 degrees C maximum;
- LubCos H2Oplus II, available from Argo Hytos, Bowling Green, Ohio, which measures dielectric constant, temperature, conductivity, and relative humidity, which is capable of < 10 bar and 85 degrees C maximum, and which is compatible with a CAN or RS232 interface;
- Condition monitor particle counter / detector model number IPDR12115240, available from Parker, Cleveland, Ohio, which measures relative humidity and ferromagnetic wear particles, which is capable of 40 to 140 ml/min (60 ml/min optimum), 2 to 420 bar, and 5 degrees C to 80 degrees C, and which is compatible with a CAN or RS232 interface;
- Fluid Property Sensor model number FPS2000, available from Parker, Cleveland, Ohio, which measures viscosity, density, dielectric constant, and temperature, which is capable of 25 bar maximum and -40 degrees C to 150 degrees C, and which is compatible with a CAN interface; and
- Moisture Sensor model number FG-K16949-KW, available from Parker, Cleveland, Ohio, which measures relative humidity, which is capable of 0.2 m/s minimum flow, 10 bar maximum, and -40 degrees C to 100 degrees C, and which is compatible with a CAN, RS232, or analogue interface.

While embodiments of the present invention are illustrated as applicable to the electro hydraulic remote connections of an agricultural tractor as used to provide hydraulic pressure and flow to the hydraulic circuits of an agricultural implement, as noted previously, it is equally contemplated that the principles of embodiments of the present invention may be applied to other agricultural, industrial, commercial, or construction vehicles or equipment that are used in hydraulic connection with removably connectable implements, devices, or attachments. As non-limiting examples, particular applications of embodiments of the present invention may include in addition to the electro hydraulic remote connections of agricultural tractors, quick connectors for the front hydraulic power lift on agricultural tractors, quick connectors for headers on combine and forage harvesters, and extra consumer hydraulic connections on excavators for hammer, crusher, rotating grapple, or rotating crusher attachments.

The invention in one form is directed to a hydraulic system for a work vehicle and at least one implement that includes at least one connection for connecting the hydraulic circuits of the work vehicle to hydraulic circuits of the at least one implement and at least one hydraulic oil quality and/or viscosity sensor. The at least one hydraulic oil quality and/or viscosity sensor is located within the hydraulic circuits of the work vehicle or within the hydraulic circuits of the at least one implement. The at least one hydraulic oil quality and/or viscosity sensor provides at least one signal based on at least one measured parameter of interest of hydraulic oil returning from or in the hydraulic circuits of the at least one implement.

The invention in another form is directed to a work vehicle having a chassis and a hydraulic system for the work vehicle and at least one implement. The hydraulic system includes at least one connection for connecting the hydraulic circuits of the work vehicle to hydraulic circuits of the at least one implement and at least one hydraulic oil quality and/or viscosity sensor. The at least one hydraulic oil quality and/or viscosity sensor is located within the hydraulic circuits of the work vehicle or within the hydraulic circuits of the at least one implement. The at least one hydraulic oil quality and/or viscosity sensor provides at least one signal based on at least one measured parameter of interest of hydraulic oil returning from or in the hydraulic circuits of the at least one implement.

An advantage of the system or arrangement of hydraulic circuits according to various embodiments of the present invention is that it limits or prevents deteriorated, contaminated, or otherwise degraded hydraulic oil within an implement, device, or attachment from intermixing significantly with the hydraulic oil of an agricultural tractor or other agricultural, industrial, commercial, or construction vehicle. Another advantage of the system or arrangement of hydraulic circuits according to embodiments of the present invention is that it may use a hydraulic oil quality and/or viscosity sensor that measures and reacts to any of a number of parameters of interest, such as viscosity, dielectric constant, density, conductivity, relative humidity, moisture content, presence of metal or other contaminants, chemical makeup, temperature, and/or pressure. Another advantage of the system or arrangement of hydraulic circuits according to embodiments of the present invention is that the at least one hydraulic oil quality and/or viscosity sensor can quickly communicate the one or more measured parameters of the implement hydraulic oil to the operator and/or control system, so that appropriate action may be taken, such as closing valves on the connectors.

Additional features and advantages of the invention will be made apparent from the following detailed description of illustrative embodiments that proceeds with reference to the accompanying drawings.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a graphical representation of a measured parameter of interest, such as viscosity, of hydraulic oil within the hydraulic circuits of an agricultural tractor before and after connection of the agricultural tractor to an agricultural implement according to the prior art;
Fig. 2 is a graphical representation of a measured parameter of interest, such as viscosity, of hydraulic oil within the hydraulic circuits of an agricultural tractor before connection of the agricultural tractor to an agricultural implement, along with avoidance of a degraded condition of the hydraulic oil within the hydraulic circuits of the agricultural tractor after connection of the agricultural tractor to the agricultural implement, according to an embodiment of the invention; and
Fig. 3 is a graphical representation of various embodiments of the present invention.

Corresponding reference characters indicate corresponding parts throughout the several views. The exemplifications set out herein illustrate embodiments of the invention, and such exemplifications are not to be construed as limiting the scope of the invention in any manner.

### DETAILED DESCRIPTION OF THE INVENTION

The term "agricultural tractor" is used herein for convenience, but should not be construed as limiting, as embodiments of the present invention are contemplated as encompassing any agricultural, industrial, commercial, or construction vehicle or machine to which the general principles of the disclosure may apply. The term "work vehicle" encompasses any work vehicle such as any agricultural, industrial, commercial, and construction work vehicle. The term "implement" includes any implement, device, or attachment which may be coupled to a work vehicle.

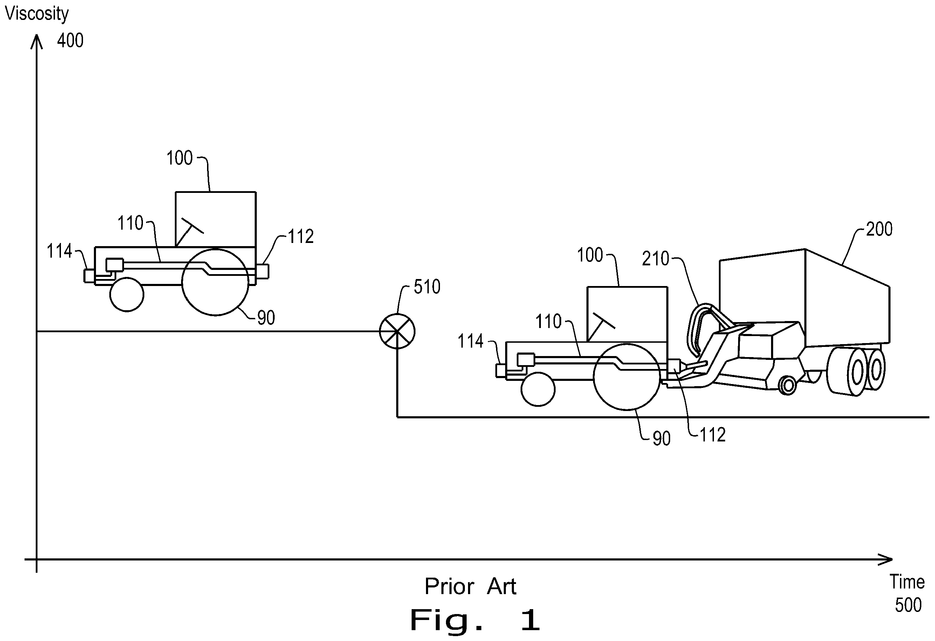

Referring now to the drawings, and more particularly to Fig. 1, there is shown a graphical representation of a parameter of interest, in this case viscosity 400, of the hydraulic oil within the hydraulic circuits 110 of an agricultural vehicle 100 having a chassis 90, measured over a period of time 500, according to the prior art. At a point in time 510, the hydraulic circuits 210 of an agricultural implement 200 are connected to the hydraulic circuits 110 of the agricultural vehicle 100 by way of electro hydraulic remote connections 112. The hydraulic circuits 110 of the agricultural vehicle 100 may be provided with a similar set of front quick connectors 114 for use with a front mounted agricultural implement, such as a loader or blade (not shown). Upon connection of the hydraulic circuits 210 of the agricultural implement 200 to the hydraulic circuits 110 of the agricultural vehicle 100 by way of the electro hydraulic remote connections 112, the hydraulic oil within the hydraulic circuits 210 of the agricultural implement 200 intermixes with the hydraulic oil within the hydraulic circuits 110 of the agricultural vehicle 100, resulting in this instance in a reduction of the viscosity of the hydraulic oil within the hydraulic circuits 110 of the agricultural vehicle 100.

This general principle holds for any given parameter of interest of the hydraulic oil within the hydraulic circuits 110 of the agricultural vehicle 100 upon connection of the hydraulic circuits 210 of the agricultural implement 200 to the hydraulic circuits 110 of the agricultural vehicle 100 by way of the electro hydraulic remote connections 112. That is to say, connection of the hydraulic circuits 210 of the agricultural implement 200 to the hydraulic circuits 110 of the agricultural vehicle 100 may result in an immediate detrimental effect to any parameter of interest, such as viscosity, dielectric constant, density, conductivity, relative humidity, moisture content, presence of metal or other contaminants, and/or chemical makeup of the hydraulic oil within the hydraulic circuits 110 of the agricultural vehicle 100. This detrimental effect therefore occurs before the operator of the agricultural vehicle 100 can be aware of it, with the potential result of having to replace the hydraulic oil within the hydraulic circuits 110 of the agricultural vehicle 100, resulting in expensive materials, labor, and downtime, or even permanent damage to the hydraulic circuits 110 of the agricultural vehicle 100.

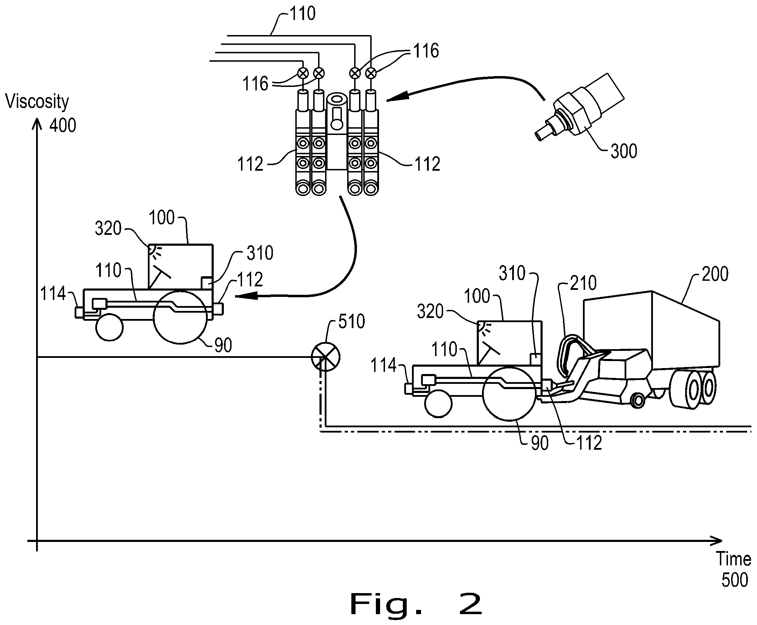

Turning, therefore, to Fig. 2, there is shown a graphical representation of a measured parameter of interest, in this case viscosity 400, of the hydraulic oil within the hydraulic circuits 110 of an agricultural vehicle 100 having a chassis 90, measured over a period of time 500, according to an embodiment of the present invention. At a point in time 510, the hydraulic circuits 210 of an agricultural implement 200 are again connected to the hydraulic circuits 110 of the agricultural vehicle 100 by way of electro hydraulic remote connections 112. The hydraulic circuits 110 of the agricultural vehicle 100 may be provided with a similar set of front quick connectors 114 for use with a front mounted agricultural implement, such as a loader or blade (not shown). However, in the embodiment of the present invention shown in Fig. 2, the hydraulic circuits 110 of the agricultural vehicle 100 are provided with at least one hydraulic oil quality and/or viscosity sensor 300 located proximate to the electro hydraulic remote connections 112. The hydraulic circuits 110 of the agricultural vehicle 100 may further be provided with at least one connector valve 116, also located proximate to or integrated with the electro hydraulic remote connections 112.

In the context of embodiments of the present invention, placing the at least one hydraulic oil quality and/or viscosity sensor 300, and/or the at least one connector valve 116, proximate to the electro hydraulic remote connections 112 of the hydraulic circuits 110 of the agricultural vehicle 100, may be defined as placing the at least one hydraulic oil quality and/or viscosity sensor 300, and/or the at least one connector valve 116 sufficiently close to the electro hydraulic remote connections 112 that upon detection of a detrimental condition as to any aforementioned measured parameter of interest within the hydraulic oil coming from the hydraulic circuits 210 of the agricultural implement 200, embodiments of the present invention may alert an operator of the agricultural vehicle 100 as to the detrimental condition and/or close the at least one connector valve 116, before any significant degradation or contamination of the hydraulic oil within the hydraulic circuits 110 of the agricultural vehicle 100 occurs. More specifically, in this embodiment, the sensor is positioned in the hydraulic circuit 110 on the agricultural vehicle 100 at a location where hydraulic oil of the implement 200 is sure to flow such that the sensor 300 determines various conditions of the hydraulic oil coming from the implement 200 instead of the hydraulic oil in the agricultural vehicle 100. For example, the sensor 300 may be positioned in the hydraulic line of the connector valve 116 to the tank. Alternatively, for example, the sensor 300 may be disposed within the hydraulic circuit 110 of the agricultural vehicle 100 and located proximate to the connections 112 within the hydraulic circuits 110 of the agricultural vehicle 100 to the hydraulic circuits 210 of the agricultural implement 200. For example, the sensor 300 may be positioned in the hydraulic oil return line within the hydraulic circuits 110 of the agricultural vehicle 100. Significant degradation or contamination may be defined as degradation or contamination of the hydraulic oil which would require the replacement of the hydraulic oil within the hydraulic circuits 110 of the agricultural vehicle 100 in order to avoid undue wear or damage to the hydraulic circuits 110.

In order to accomplish this, the at least one hydraulic oil quality and/or viscosity sensor 300, and/or the at least one connector valve 116, may be connected to an electronic control unit (ECU) 310 and/or an alarm, indicator, or display 320. The ECU 310 may interpret the signals of the at least one hydraulic oil quality and/or viscosity sensor 300, and may further send signals, commands, or instructions to either or both of the alarm, indicator, or display 320 and the at least one connector valve 116. Specifically, upon interpretation by the ECU 310 that the at least one hydraulic oil quality and/or viscosity sensor 300 has detected a detrimental condition as to any aforementioned parameter of interest within the hydraulic oil coming from the hydraulic circuits 210 of the agricultural implement 200, or in other words that the one or more measured parameters of the hydraulic oil compares unfavorably with one or more standard parameters for hydraulic oil, one or more thresholds of acceptability for the one or more measured parameters, and/or known or measured parameters of the hydraulic oil, the ECU 310 may instruct the alarm, indicator, or display 320 to notify an operator of the agricultural vehicle 100, and/or may command the at least one connector valve 116 to close.

Alternate embodiments of the present invention may be arranged so that the ECU 310 commands the at least one connector valve 116 to close unless overridden by the operator, or may command the at least one connector valve 116 to close upon verification by the operator. Still further alternate embodiments of the present invention may be arranged so that the at least one connector valve 116 remains closed upon connection of the agricultural implement 200 to the agricultural 100 by way of the electro hydraulic remote connections 112 until such time that the at least one hydraulic oil quality and/or viscosity sensor (300) determines that the one or more of parameters of interest are within acceptable limits. In any case, significant degradation or contamination of the hydraulic oil within the hydraulic circuits 110 of the agricultural vehicle 100 is avoided following the point in time 510 at which the hydraulic circuits 210 of the agricultural implement 200 are connected to the hydraulic circuits 110 of the agricultural vehicle 100 by way of the electro hydraulic remote connections 112, as represented by the phantom line representing viscosity 400 following the point in time 510.

In an alternative embodiment, the at least one hydraulic oil quality and/or viscosity sensor 300 is positioned in the hydraulic circuit 210 of the agricultural implement 200. The signals of the at least one hydraulic oil quality and/or viscosity sensor 300 can be sent to the ECU 310 as soon as the agricultural vehicle 100 is connected, for example via an ISOBUS connection. It is also conceivable to wirelessly forward the signals of the at least one hydraulic oil quality and/or viscosity sensor 300 to the agricultural vehicle 100 as soon as the operator connects or intends to couple the agricultural implement 200 to the agricultural vehicle 100.

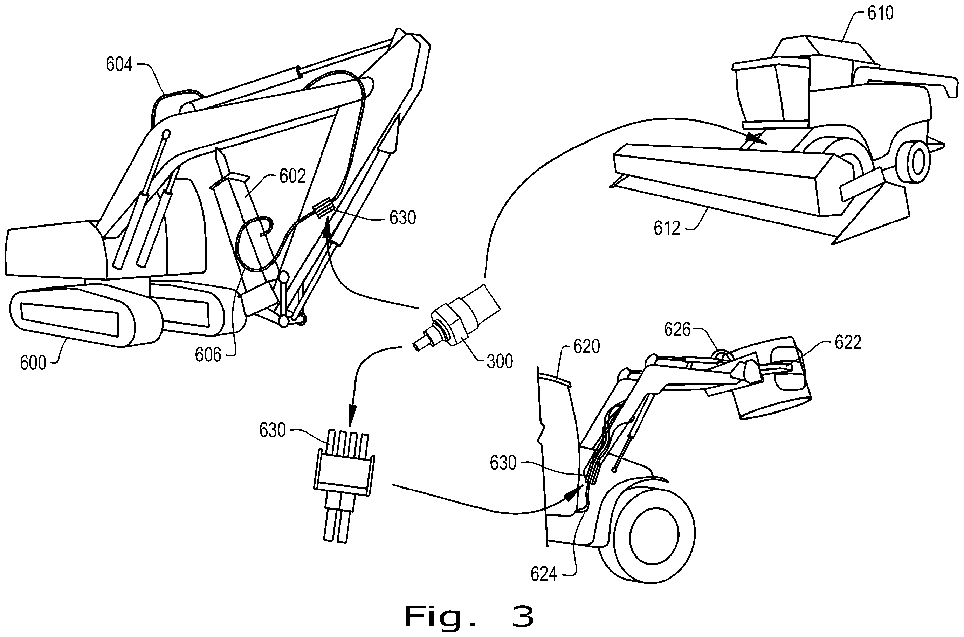

Turning now to Fig. 3, various embodiments of the present invention are shown, indicating applications in which the present invention may be used. In exemplary illustration, an excavator 600 has hydraulic circuits 604, to which are connected the hydraulic circuits 606 of an excavator attachment 602 by way of quick connectors 630. At least one hydraulic oil quality and/or viscosity sensor 300 is connected to the hydraulic circuits 604 of the excavator 600 proximate to the quick connectors 630, according to an embodiment of the present invention. Similarly, an agricultural tractor 620 has hydraulic circuits 624, to which are connected the hydraulic circuits 626 of a loader attachment 622 by way of quick connectors 630. Again, at least one hydraulic oil quality and/or viscosity sensor 300 is connected to the hydraulic circuits 624 of the agricultural tractor 620 proximate to the quick connectors 630, according to an embodiment of the present invention. An agricultural harvester 610 is also illustrated which may employ an embodiment of the present invention in its hydraulic connections (not visible) to header 612. In this way, significant degradation or contamination of the hydraulic oil within the hydraulic circuits 604, 624 of the excavator 600, agricultural harvester 610, or agricultural tractor 620 is avoided when the hydraulic circuits 606, 626 of the excavator attachment 602, header 612, or loader attachment 622, respectively, are connected to the hydraulic circuits 604, 624 of the excavator 600, agricultural harvester 610, or agricultural tractor 620, either by alerting an operator to a detrimental condition as to any aforementioned parameter of interest within the hydraulic oil coming from the hydraulic circuits 606, 626 of the excavator attachment 602, header 612, or loader attachment 622, or by closure of one or more connector valves (not shown) at the quick connectors 630, or both.

## Claims

1. A hydraulic system for a work vehicle (100), comprising:
at least one connection (112) for connecting hydraulic circuits (110) of the work vehicle (100) to a hydraulic circuit (210) of one implement (200); and
at least one hydraulic oil quality sensor (300);
said at least one sensor (300) being located within said hydraulic circuits (110) of the work vehicle (100) and said at least one sensor (300) providing at least one signal based on at least one measured parameter of interest of hydraulic oil flowing from the hydraulic circuit (210) of said implement (200) when attached to said work vehicle (100),
**characterized in that:**
said system further comprises at least one connector valve (116) being operable to close the oil flow coming from the hydraulic circuit (210) of said implement (200) when said sensor (300) measures a detrimental condition of said at least one parameter of interest such that significant degradation or contamination of the hydraulic oil within the hydraulic circuits (110) of the work vehicle (100) is prevented.

2. A hydraulic system for a work vehicle (100) according to claim 1, wherein said sensor is located proximate to said at least one connection (112) within said hydraulic circuits (110) of the work vehicle (100).

3. The hydraulic system of claim 1 or 2, wherein:
said at least one measured parameter of interest further includes at least one of viscosity, dielectric constant, density, conductivity, relative humidity, moisture content, presence of contaminants, chemical makeup.

4. The hydraulic system of claim 1 or 2, further comprising:
at least one electronic control unit (310) connected to said sensor (300) and configured to compare said at least one measured parameter of interest to at least one of:
at least one standard parameter for hydraulic oil;
at least one absolute or comparative threshold of acceptability for hydraulic oil; and
at least one known or measured parameter of hydraulic oil within the hydraulic circuits (110) of the work vehicle (100).

5. The hydraulic system of any claim from 1 to 4, further comprising:
at least one alarm, indicator, or display (320) directly or indirectly connected to said sensor (300) and configured to notify an operator of the work vehicle (100) of said at least one measured parameter of interest.

6. The hydraulic system of claim 5, wherein:
said at least one alarm, indicator, or display (320) being further configured to convey at least one instruction to the operator as to an appropriate action to be taken.

7. The hydraulic system of any claim from 1 to 4, wherein:
said at least one connector valve (116) being operable to at least one of:
close upon said sensor (300) measuring a detrimental condition of said at least one parameter of interest, unless overridden by an operator;
close upon said sensor (300) measuring a detrimental condition of said at least one parameter of interest and upon verification by an operator;
remain closed by default upon connection of the at least one implement (200) to the work vehicle (100), until said sensor (300) measures at least one parameter of interest that is within acceptable limits.

8. The hydraulic system of any claim from 1 to 7, wherein:
said at least one connection (112) for connecting the hydraulic circuits (110) of the work vehicle (100) to hydraulic circuits (210) of the at least one implement (200) further comprises at least one electro hydraulic remote connection (112).

9. The hydraulic system of any claim from 1 to 8, wherein:
said sensor (300) is located within said hydraulic circuits (110) of the work vehicle (100) such that said sensor (300) is positioned in the oil return line.

10. A work vehicle (100) comprising the hydraulic system of any one of the preceding claims.

## Patentansprüche

1. Hydrauliksystem für ein Arbeitsfahrzeug (100) mit:
mindestens einer Verbindung (112) zum Verbinden von Hydraulikkreisen (110) des Arbeitsfahrzeugs (100) mit einem Hydraulikkreis (210) eines Arbeitsgeräts (200);
und mindestens einem Hydrauliköl-Qualitätssensor (300);
wobei der mindestens eine Sensor (300) sich innerhalb der Hydraulikkreise (110) des Arbeitsfahrzeugs (100) befindet und der mindestens eine Sensor (300) mindestens ein Signal bereitstellt, das auf mindestens einem gemessenen Parameter von Interesse des Hydrauliköls basiert, das von dem Hydraulikkreis (210) des Arbeitsgeräts (200) abfließt, wenn es an dem Arbeitsfahrzeug (100) angebracht ist,
**dadurch gekennzeichnet, dass**:
das System weiterhin mindestens ein Verbindungsventil (116) umfasst, das betreibbar ist, um den Ölfluss, der von dem Hydraulikkreis (210) des Arbeitsgeräts (200) kommt, zu schließen, wenn der Sensor (300) einen nachteiligen Zustand von mindestens einem Parameter von Interesse erfasst, sodass eine wesentliche Verschlechterung oder Kontaminierung des Hydrauliköls innerhalb des Hydraulikkreises (110) des Arbeitsfahrzeugs (100) verhindert wird.

2. Hydrauliksystem für ein Arbeitsfahrzeug (100) nach Anspruch 1, wobei sich der Sensor in der Nähe der mindestens einen Verbindung (112) innerhalb der Hydraulikkreise (110) des Arbeitsfahrzeugs (100) befindet.

3. Hydrauliksystem nach Anspruch 1 oder 2, wobei:
der mindestens eine gemessene Parameter von Interesse weiterhin mindestens eines aus Viskosität, Dielektrizitätskonstante, Dichte, Leitfähigkeit, relative Feuchtigkeit, Feuchtigkeitsgehalt, Vorhandensein von Schadstoffen, chemische Zusammensetzung umfasst.

4. Hydrauliksystem nach Anspruch 1 oder 2, weiterhin aufweisend:
mindestens eine elektronische Steuereinrichtung (310), die mit dem Sensor (300) verbunden ist und dazu eingerichtet ist, den mindestens einen gemessenen Parameter von Interesse mit mindestens einem der folgenden Parameter zu vergleichen:
mindestens einem Standardparameter für Hydrauliköl;
mindestens einem absoluten oder vergleichenden Akzeptanzgrenzwert für Hydrauliköl;
mindestens einem bekannten oder gemessenen Parameter von Hydrauliköl innerhalb der Hydraulikkreisläufe (110) des Arbeitsfahrzeugs (100).

5. Hydrauliksystem nach einem der Ansprüche 1 bis 4, weiterhin aufweisend:
mindestens einen Alarmgeber, Indikator oder Bildschirm (320), der direkt oder indirekt mit dem Sensor (300) verbunden ist und dazu eingerichtet ist, den Bediener des Arbeitsfahrzeugs (100) über den mindestens einen gemessenen Parameter von Interesse zu benachrichtigen.

6. Hydrauliksystem nach Anspruch 5, wobei:
der mindestens eine Alarmgeber, Indikator oder Bildschirm (320) weiterhin dazu eingerichtet ist, mindestens eine Anweisung an den Bediener hinsichtlich einer geeigneten zu ergreifenden Maßnahme zu übermitteln.

7. Hydrauliksystem nach einem der Ansprüche 1 bis 4, wobei:
das mindestens eine Verbindungsventil (116) betätigbar ist für mindestens eine der folgenden Handlungen:
Schließen, wenn der Sensor (300) einen nachteiligen Zustand von mindestens einem Parameter von Interesse erfasst, sofern nicht von einem Bediener überschrieben;
Schließen, wenn der Sensor (300) einen nachteiligen Zustand von mindestens einem Parameter von Interesse erfasst und nach Bestätigung durch einen Bediener;
standardmäßiges Verbleiben im geschlossenen Zustand nach dem Anschluss des mindestens einen Arbeitsgeräts (200) an das Arbeitsfahrzeug (100), bis der Sensor (300) mindestens einen Parameter von Interesse erfasst, der innerhalb akzeptabler Grenzen liegt.

8. Hydrauliksystem nach einem der Ansprüche 1 bis 7, wobei:
die mindestens eine Verbindung (112) zum Verbinden der Hydraulikkreise (110) des Arbeitsfahrzeugs (100) mit Hydraulikkreisen (210) des mindestens einen Arbeitsgeräts (200) weiterhin mindestens eine elektrohydraulische Fernanschlusseinrichtung (112) aufweist.

9. Hydrauliksystem nach einem der Ansprüche 1 bis 8, wobei:
der Sensor (300) sich innerhalb der Hydraulikkreise (110) des Arbeitsfahrzeugs (100) befindet, sodass der Sensor (300) in der Ölrücklaufleitung positioniert ist.

10. Arbeitsfahrzeug (100) mit dem Hydrauliksystem nach einem der vorangehenden Ansprüche.

## Revendications

1. Système hydraulique pour véhicule de travail (100), comprenant :
au moins une connexion (112) pour connecter des circuits hydrauliques (110) du véhicule de travail (100) à un circuit hydraulique (210) d'un outil (200) ;
et au moins un capteur de qualité hydraulique de l'huile (300) ;
ledit au moins un capteur (300) étant situé dans lesdits circuits hydrauliques (110) du véhicule de travail (100) et ledit au moins un capteur (300) fournissant au moins un signal sur la base d'au moins un paramètre d'intérêt mesuré de l'huile hydraulique s'écoulant du circuit hydraulique (210) dudit outil (200) lorsqu'il est attaché audit véhicule de travail (100),
**caractérisé en ce que** :
ledit système comprend en outre au moins une valve de raccordement (116) pouvant être actionnée pour fermer la circulation d'huile provenant du circuit hydraulique (210) dudit outil (200) lorsque ledit capteur (300) mesure une condition nuisible dudit au moins un paramètre d'intérêt de sorte qu'une dégradation ou une contamination significative de l'huile hydraulique dans les circuits hydrauliques (110) du véhicule de travail (100) est empêchée.

2. Système hydraulique pour un véhicule de travail (100) selon la revendication 1, dans lequel ledit capteur est situé à proximité de ladite au moins une connexion (112) dans lesdits circuits hydrauliques (110) du véhicule de travail (100).

3. Système hydraulique selon la revendication 1 ou 2, dans lequel :
ledit au moins un paramètre d'intérêt mesuré comprend en outre au moins l'un des éléments suivants : viscosité, constante diélectrique, densité, conductivité, humidité relative, teneur en humidité, présence de contaminants, composition chimique.

4. Système hydraulique selon la revendication 1 ou 2, comprenant en outre :
au moins une unité de commande électronique (310) couplée audit capteur (300) et configurée pour comparer ledit au moins un paramètre d'intérêt mesuré à au moins l'un des éléments suivants :
au moins un paramètre standard pour l'huile hydraulique ; au moins un seuil d'acceptabilité absolu ou comparatif pour l'huile hydraulique ;
et
au moins un paramètre connu ou mesuré de l'huile hydraulique à l'intérieur des circuits hydrauliques (110) du véhicule de travail (100).

5. Système hydraulique selon l'une quelconque des revendications 1 à 4, comprenant en outre : au moins une alarme, un indicateur ou un écran (320) connecté directement ou indirectement audit capteur (300) et configuré pour notifier un opérateur du véhicule de travail (100) dudit au moins un paramètre d'intérêt.

6. Système hydraulique selon la revendication 5, dans lequel :
ledit au moins une alarme, un indicateur, ou un écran (320) étant en outre configuré pour transmettre au moins une instruction à l'opérateur quant à une action appropriée à prendre.

7. Système hydraulique selon l'une quelconque des revendications 1 à 4, dans lequel : ladite au moins une valve de raccordement (116) étant actionnée pour au moins :
fermer ledit capteur (300) mesurant une condition nuisible dudit au moins un paramètre d'intérêt, sauf s'il est annulé par un opérateur ;
fermer ledit capteur (300) mesurant une condition nuisible dudit au moins un paramètre d'intérêt et après vérification par un opérateur ;
rester fermé par défaut lors de la connexion d'au moins un outil (200) au véhicule de travail (100), jusqu'à ce que ledit capteur (300) mesure au moins un paramètre d'intérêt dans les limites acceptables.

8. Système hydraulique selon l'une quelconque des revendications 1 à 7 dans lequel : ladite au moins une connexion (112) pour connecter les circuits hydrauliques (110) du véhicule de travail (100) aux circuits hydrauliques (210) de l'au moins un outil (200) comprend en outre au moins une connexion électrohydraulique à distance (112).

9. Système hydraulique selon l'une quelconque des revendications 1 à 8, dans lequel :
ledit capteur (300) est situé à l'intérieur desdits circuits hydrauliques (110) du véhicule de travail (100) de sorte que ledit capteur (300) est situé dans la ligne de retour d'huile.

10. Véhicule de travail (100) comprenant le système hydraulique selon l'une quelconque des revendications précédentes.
